# EUROPEAN PATENT APPLICATION

(11) **EP 4 649 906 A1**
(43) Date of publication of application: **19.11.2025**
(21) Application number: 24756630.0
(22) Date of filing: 29.01.2024
(51) Int. Cl.: A61B 18/14

(54) **HIGH FREQUENCY ELECTRODE PROBE WITH TEMPERATURE MEASUREMENT FUNCTION**

(30) Priority: 16.02.2023 JP 2023022777
(71) Applicant: Kabushiki Kaisha Top, Adachi-ku, Tokyo 120-0035 (JP)
(72) Inventor: MURATA Koji, Tokyo 120-0035 (JP); MIYAZAWA Yoshihiro, Tokyo 120-0035 (JP)
(74) Representative: Schön, Christoph
(86) International application number: PCT/JP2024/002615
(87) International publication number: WO 2024/171779

(57) **Abstract**

Provided is a high frequency electrode probe with a temperature measurement function, in which a thermocouple and a compensation conductive wire can be connected to each other without using a connection joint, or the compensation conductive wire is not required, and provided is a high frequency electrode probe with a temperature measurement function, in which an alignment between the connection joint and an orientation of a cutting edge surface of a probe tube is not required, and the cutting edge surface of the probe tube can be easily aligned with a probe proximal cover. The high frequency electrode probe with a temperature measurement function includes a cylindrical probe tube 11, a thermocouple including a pair of thermocouple conductive wires W1+ and W1- electrically connected at a temperature measurement contact point C1 disposed at a distal end portion of the probe tube 11, and a high frequency conductive wire WO electrically connected to the probe tube 11, in which a stopper tube 12 having flexibility is externally attached to a proximal end portion of the probe tube, and at least the thermocouple conductive wires W1+ and W1- are sandwiched between an outer peripheral surface of the probe tube l1 and an inner peripheral surface of the stopper tube 12 or sandwiched between the outer peripheral surface of the probe tube 11 and an inner peripheral surface of a medicinal liquid tube 16.

## Description

### Technical Field

The present invention relates to a high frequency electrode probe having a temperature measurement function, which can coagulate a specific tissue by emitting a high frequency current and/or performing a nerve block by administering a pulsed high frequency and/or an anesthetic solution from a probe distal portion.

### Background Art

As a device for a nerve block for pain relief treatment, a high frequency electrode probe having a drug injection function such as an anesthetic and a tissue coagulation or cauterization function by applying electric energy is known. The high frequency electrode probe is normally provided with a temperature sensor that measures the temperature of the probe distal part in order to control electric energy. A thermocouple is used for the temperature sensor because the thermocouple is required to be relatively small in order to be disposed inside a probe tube.

In general, since a thermocouple conductive wire is expensive and has problems of resistance value, strength, and elasticity, it is rare to stretch the thermocouple conductive wire to the length equal to or longer than a predetermined length. Therefore, the thermocouple conductive wire is connected to a compensation conductive wire via a relay member, and is connected to a device that transmits a temperature measurement signal, such as a control device.

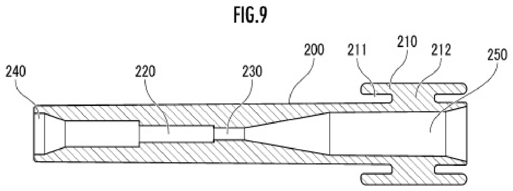

As such a relay member, for example, a connection joint 200 that also functions as a probe proximal part as illustrated in FIG. 9 is known. The connection joint 200 in the illustrated example is a hollow cylindrical member, and an internal space penetrates from a proximal end to a distal end. In addition, a pair of winding portions 210 that locally protrude in the radial direction is formed on the outer periphery of a proximal end portion. In the winding portion 210, a recessed portion 211 is formed around a proximal portion 212. Each of the thermocouple conductive wires and the compensation conductive wire corresponding to each of the thermocouple conductive wires are wound around the recessed portion 211 a plurality of times (for example, four times) to be fixed, and the thermocouple conductive wires and the compensation conductive wires are electrically connected to each other.

A large-diameter medicinal liquid tube insertion portion 250 is formed on the inner periphery on the proximal end side having the winding portion 210, and the medicinal liquid tube is inserted and fixed to the medicinal liquid tube insertion portion 250 in a liquid-tight manner. A probe tube insertion portion 240 is formed on a distal end side of the connection joint 200, and a probe tube fixing portion 220 is further formed on a proximal end side of the connection joint 200. A small diameter portion 230 for constituting a fixing structure of a probe tube proximal end portion called a cannula stop, which regulates a proximal end position of the probe tube and defines an effective length of the probe tube, is formed on a proximal end side of the probe tube fixing portion 220, and a proximal end surface of the probe tube abuts on a boundary between the probe tube fixing portion 220 and the small diameter portion 230 such that a position of the probe tube in the longitudinal direction is regulated. The medicinal liquid tube and the probe tube are fluidically communicated through an internal space of the connection joint 200.

However, since the work of winding the wire around the connection joint 200 is normally performed manually, the work efficiency is poor, which is a factor that significantly reduces productivity. In addition, the probe tube fixing portion 220 needs to be set to an inner diameter corresponding to the size of the probe tube to be fixed. In a case where the probe tubes having a plurality of sizes are used, core pins for setting the inner diameters as many as the number corresponding to the probe tubes are required, which complicates the manufacturing tools and increases the cost. Furthermore, since the vicinity of the small diameter portion 230 is thick, which causes a molding defect called a sink mark.

In addition, when the connection joint 200 and the proximal end portion of the probe tube are fixed, it is necessary to align the connection joint 200 and the orientation of a cutting edge surface of the probe tube in order to improve workability during the medical treatment, but this alignment work is not easy and makes the assembly work even more difficult.

Patent Literature 1 (PCT Japanese Translation Patent Publication No. 2018-525180) discloses a treatment apparatus including a probe device, a suction device that is attached to and detached from the probe device for tissue collection, an injection device that is attached to and detached from the probe device for drug injection, and an electrode device that is attached to and detached from the probe device for thermal treatment.

The treatment apparatus controls the power applied to a power cable according to a temperature measured by a temperature sensor located at a probe tip portion T before the thermal treatment is performed. A sensing cable that connects the temperature sensor and the control device penetrates the inside of the power cable and protrudes from an end portion of the power cable. Therefore, it is necessary to integrate the power cable and the sensing cable. However, for example, in a case where a thermocouple is used as the temperature sensor, it is necessary to form a sensing cable 43 with a thermocouple strand or a compensation conductive wire, and it is difficult to integrate such a sensing cable and a power cable for flowing an RF current. Furthermore, this document does not disclose details of a method of fixing the power cable and the injection cable.

Patent Literature 2 (PCT Japanese Translation Patent Publication No. 2015-522358) discloses a catheter having a handle assembly including an electrical connector port and a fluid connector port. However, the electrical connector port of this document is positioned at a proximal end portion of the handle assembly itself and includes only a cylindrical passage that penetrates a part of the main body. In addition, it is disclosed that the electrical wire may be electrically coupled to an external electrical connection portion, and the external electrical connection portion may be inserted into a proximal internal handle part through the electrical connector port. However, there is no specific description of a method of connecting or fixing the thermocouple conductive wire and the compensation conductive wire or the high frequency conductive wire.

Patent Literature 3 (Japanese Patent Application Laid-Open No. 2011-136173) discloses a medical probe including an ablation electrode, a first conductor that is connected to the ablation electrode and is configured to transmit ablation energy to the ablation electrode, and a second conductor that is connected to the first conductor at a contact point to form a thermocouple at the contact point. In addition, the fact that high frequency (RF) energy is supplied to the ablation electrode fixed at the end point of the flexible insertion tube constituting the probe, and the fact that the thermocouple is located in the tube at a distal end are disclosed. The fact that the thermocouple includes a first conductor and a second conductor, the two conductors are galvanically connected to each other at the contact point, the two conductors are selected to be different from each other, the two conductors are selected from known components having a known thermocouple voltage-temperature relationship, the first conductor includes copper, and the second conductor 42 includes a copper-nickel alloy such as constantan, and the fact that the contact point is also electrically connected to the ablation electrode are disclosed.

However, although this document uses a conductive wire, which is one of the thermocouple conductive wires, to supply the high frequency power, the power supply point is not the proximal end of the probe tube but a contact point of the thermocouple conductive wire at the distal end of the tube. In addition, this document does not disclose any specific connection method or fixing method for the thermocouple conductive wire and the compensation conductive wire, or any specific connection method or fixing method for the high frequency conductive wire, similar to Patent Literature 2.

### Citation List

### Patent Literatures

Patent Literature 1: PCT Japanese Translation Patent Publication No. 2018-525180
Patent Literature 2: PCT Japanese Translation Patent Publication No. 2015-522358
Patent Literature 3: Japanese Patent Application Laid-Open No. 2011-136173

### Summary of Invention

### Technical Problem

It is difficult to solve the above-described problem when a configuration is adopted in which the thermocouple conductive wire and the compensation conductive wire are fixed and electrically connected to each other via the connection joint that also functions as a probe proximal part. In addition, the above documents 1 to 3 also make no mention of a connection joint that connects the thermocouple conductive wire and the compensation conductive wire. Furthermore, in recent years, a semiconductor element such as a thermistor is often used as a temperature measurement element, and there is a concern that the supply of some types of compensation conductive wires may be delayed due to a decline in demand for thermocouple elements.

In addition, when fixing the connection joint and the proximal end portion of the probe tube, it is necessary to align the connection joint and the orientation of the cutting edge surface of the probe tube, which makes the assembly work even more difficult.

Therefore, an object of the present invention is to provide a high frequency electrode probe with a temperature measurement function, in which a thermocouple and a compensation conductive wire can be connected to each other without using a connection joint, or the compensation conductive wire is not required.

In addition, an object of the present invention is to provide a high frequency electrode probe with a temperature measurement function, in which an alignment between a connection joint and an orientation of a cutting edge surface of a probe tube is not required, and the cutting edge surface of the probe tube can be easily aligned with a probe proximal cover serving as a handle.

### Solution to Problem

The present invention relates to a high frequency electrode probe with a temperature measurement function (hereinafter, may be referred to as a "high frequency electrode probe" for simplification of expression), including a cylindrical probe tube, a thermocouple including a pair of thermocouple conductive wires electrically connected at a temperature measurement contact point disposed at a distal end portion of the probe tube, and a high frequency conductive wire electrically connected to the probe tube, in which a stopper tube having flexibility is externally attached to a probe tube proximal end portion serving as a proximal end portion of the probe tube, and the thermocouple conductive wire is sandwiched between an outer peripheral surface of the probe tube and an inner peripheral surface of the stopper tube.

Alternatively, the present invention may have a configuration in which a cylindrical probe tube, a thermocouple including a pair of thermocouple conductive wires electrically connected at a temperature measurement contact point disposed at a distal end portion of the probe tube, a high frequency conductive wire electrically connected to the probe tube, and a medicinal liquid tube configured to supply a medicinal liquid to the probe tube are provided, and at least the thermocouple conductive wire is sandwiched between an outer peripheral surface of the probe tube and an inner peripheral surface of the medicinal liquid tube.

In the high frequency electrode probe according to the present invention, since a stopper tube is disposed at the probe tube proximal end portion, and a pair of thermocouple conductive wires led out from the probe tube are sandwiched between the inner peripheral surface of the medicinal liquid tube and the outer peripheral surface of the probe tube, the thermocouple conductive wires are fixed, and thus a connection joint is not required. The connection between the thermocouple conductive wire and the compensation conductive wire may be performed using known means for connecting electrical wires to each other.

In addition, the present invention preferably includes a probe proximal cover configured to enclose the probe tube proximal end portion and serve as a handle of the probe tube at the probe tube proximal end portion, and an indexing mechanism configured to mechanically regulate a circumferential position of a cutting edge surface of the probe tube with respect to the probe proximal cover.

The probe proximal cover serving as a handle is provided, so that the high frequency electrode probe is easily operated. Furthermore, since the probe proximal cover has an indexing mechanism configured to mechanically regulate the circumferential position of the cutting edge surface of the probe tube with respect to the probe proximal cover, the position of the cutting edge surface can be easily aligned with a circumferential specific position of the probe proximal cover, and the position of the cutting edge surface can be easily confirmed at hand, so that the efficiency of the hand technique is improved. In this case, a mark indicating the position of the cutting edge surface may be provided at the circumferential specific position of the probe proximal cover.

In addition, the indexing mechanism may be a fitting mechanism of a protrusion structure formed at a circumferential fixed position of the probe tube and a recessed structure formed at a fixed position on the inner peripheral surface of the probe proximal cover. By making the indexing mechanism such a fitting mechanism, the position of the cutting edge surface can be easily aligned with the circumferential specific position of the probe proximal cover with a simple structure.

Furthermore, the protrusion structure may be configured such that a metal pipe formed in a crank shape or a Z shape is fixed to a fixed position on a peripheral surface of the probe tube, and the high frequency conductive wire is crimped and connected within the metal pipe of the protrusion structure and is electrically conductive to the probe tube via the metal pipe.

By forming the protrusion structure with the metal pipe, the protrusion structure can be easily formed only by bending the metal pipe and fixing means without processing the probe tube. As the fixing means, known connection means between metals may be used. In addition, by using the metal pipe for connecting the high frequency conductive wire, the high frequency conductive wire is inserted into the metal pipe, and the metal pipe is crushed, thereby the high frequency conductive wire and the metal pipe can be crimped to be electrically connected. Furthermore, since the metal pipe receives a stress load when the probe tube is inserted into the probe proximal cover, the risk of disconnection of the high frequency conductive wire is reduced, and the quality can be stabilized.

In the present invention, the high frequency conductive wire may be sandwiched between the outer peripheral surface of the probe tube and the inner peripheral surface of the stopper tube, and be crimped and connected to the probe tube to be electrically conductive, without using the metal pipe. Alternatively, the high frequency conductive wire may be sandwiched between the outer peripheral surface of the probe tube and the inner peripheral surface of the medicinal liquid tube, and be electrically conductive by being crimped and connected to the probe tube.

As described above, similar to the thermocouple conductive wire, the high frequency conductive wire between the stopper tube or the medicinal liquid tube and the probe tube are sandwiched and fixed. Therefore, the high frequency conductive wire can be fixed with a simple configuration, and the electrical conduction of the two can be achieved. That is, since the insulating member is not present on the outer surface of the high frequency conductive wire and the probe tube in the fixing portion, the metals of the two are crimped, and the electrical conduction can be easily performed without passing through a troublesome step for electrical connection such as soldering.

In the present invention, the thermocouple conductive wire may extend to and be directly electrically connected to a thermocouple connector for connecting to a high frequency power supply device without passing through a compensation conductive wire.

As described above, by extending the thermocouple conductive wire to the thermocouple connector without using the compensation conductive wire and directly connecting the thermocouple conductive wire to the thermocouple connector, it is possible to manufacture the high frequency electrode probe even in a case where the supply of the compensation conductive wire is delayed. In this case, a pair of thermocouple conductive wires of a required length suitable for measuring a desired temperature, and end portions of the two are connected to form a temperature measurement contact point, which may be used as a thermocouple.

Alternatively, in the present invention, the thermocouple conductive wire may be connected to the compensation conductive wire in the vicinity of the stopper tube by a crimp connector, and the compensation conductive wire may extend to a thermocouple connector for connecting a high frequency generator and be directly electrically connected to the thermocouple connector. In addition, in this case, the high frequency conductive wire may be connected by the crimp connector to one of the compensation conductive wires together with one of the thermocouple conductive wires.

By connecting the thermocouple conductive wire and the compensation conductive wire with the crimp connector, the two can be easily electrically connected to each other, and the working time can be shortened. In addition, when one material of the compensation conductive wire is a material suitable for transmitting high frequency power, such as copper, the high frequency conductive wire can be connected to the compensation conductive wire to function as a high frequency conductive wire. As a result, the number of wirings to the thermocouple connector can be reduced by one, and cost reduction and simplification of the structure can be achieved.

In the present invention, a configuration may be adopted in which a positioning tube is externally attached to the proximal end portion of the probe tube, and a distal end portion of the positioning tube is locked to a stepped portion in the probe proximal cover to regulate an axial position of the probe tube.

By forming a cannula stop structure that regulates the position of the probe tube proximal end portion by the positioning tube distal end portion and the stepped portion in the probe proximal cover and defines the effective length of the probe tube, the connection joint in which the cannula stop was formed in the related art is not required, and the problem of molding defect called a sink mark due to a thick portion in the vicinity of the cannula stop is also solved.

### Brief Description of Drawings

FIG. 1 is a diagram illustrating a configuration of a high frequency electrode probe as one embodiment of the present invention.
FIG. 2 is a detailed diagram illustrating a configuration of a distal end portion of a probe tube (X1 part in FIG. 1).
FIG. 3 is a detailed diagram illustrating a configuration of the distal end portion of the probe tube (X1 part in FIG. 1).
FIG. 4 is a detailed diagram illustrating a configuration of a thermocouple connector (X3 part in FIG. 1).
FIG. 5 is a diagram illustrating a configuration of a high frequency electrode probe as a second embodiment of the present invention (X2 part in FIG. 1).
FIG. 6 is a diagram illustrating a configuration of a probe proximal cover as viewed from a distal end (probe tube disposition side).
FIG. 7 is a diagram illustrating a configuration of a high frequency electrode probe as a third embodiment of the present invention (X2 part in FIG. 1).
FIG. 8 is a diagram illustrating a configuration of a high frequency electrode probe of a modification example in FIG. 7 (X2 part in FIG. 1).
FIG. 9 is a diagram illustrating a configuration example of a probe proximal part in the related art.

### Description of Embodiments

A high frequency electrode probe with a temperature measurement function according to the present invention includes a cylindrical probe tube 11, a thermocouple 14 including a pair of thermocouple conductive wires W1+ and W1- electrically connected at a temperature measurement contact point C1 disposed at a distal end portion of the probe tube 11, and a high frequency conductive wire WO electrically connected to the probe tube 11, in which a stopper tube 12 having flexibility is externally attached to a probe tube proximal end portion 11a of the probe tube 11, and at least the thermocouple conductive wires W1+ and W1- are sandwiched between an outer peripheral surface of the probe tube 11 and an inner peripheral surface of the stopper tube 12. In addition, in a case where the thickness of the probe tube 11 is larger than the inner diameter of a medicinal liquid tube 16, the medicinal liquid tube 16 may be responsible for the function instead of the stopper tube 12.

### (Configuration of High Frequency Electrode Probe)

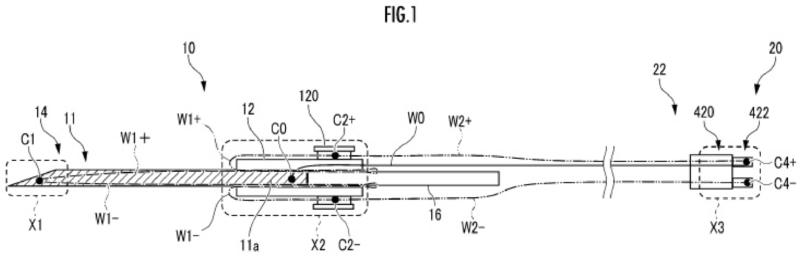

Next, a more specific configuration of the high frequency electrode probe according to the present invention will be described with reference to the drawings. The high frequency electrode probe according to the first embodiment of the present invention schematically illustrated in FIG. 1 includes the substantially cylindrical probe tube 11, the substantially cylindrical stopper tube 12 externally attached to the proximal end portion of the probe tube 11, the high frequency conductive wire WO (refer to solid line) electrically connected to the probe tube 11, the thermocouple 14 including a pair of thermocouple conductive wires W1+ and W1- (refer to one-dot chain line), and a pair of compensation conductive wires W2+ and W2- (refer to two-dot chain line).

In the probe tube 11, the stopper tube 12 is externally attached to the probe tube proximal end portion 11a, which is a proximal end side of the probe tube 11. That is, the stopper tube 12 is mounted on the probe tube 11 by inserting the proximal end portion of the probe tube 11 into the inside of the stopper tube 12 or a hollow space. In this case, the inside of the probe tube 11 communicates with the outside from the proximal end portion of the stopper tube 12. A distal end portion of the medicinal liquid tube 16 is connected to a proximal end of the probe tube 11. In a case where a connection tube 13 described later is used for connecting the two, or the outer diameter of the probe tube 11 is larger than the outer diameter of the medicinal liquid tube 16, the distal end portion of the medicinal liquid tube 16 is externally attached to the proximal end portion of the probe tube 11 to connect the two. As a result, a medicinal liquid for treatment, such as an anesthetic agent, is supplied from a medicinal liquid supply device (not illustrated) connected to the proximal end portion of the medicinal liquid tube 16 to the inside of the probe tube 11 via the medicinal liquid tube 16, and the medicinal liquid can be discharged from the distal end portion of the probe tube 11.

The stopper tube 12 is made of a flexible resin material, and is formed into a substantially cylindrical shape of a predetermined length or into a cylindrical shape having an inner peripheral surface that matches the shape of the outer peripheral surface of the probe tube 11. The inner diameter of the stopper tube 12 is preferably slightly smaller than the outer diameter of the probe tube 11 to be mounted, and specifically, the difference between the inner diameter of the stopper tube 12 and the outer diameter of the probe tube 11 is preferably 0.01 mm or more, and more preferably 0.05 mm or more. The upper limit thereof depends on the material of the stopper tube 12, and it is preferable that the difference between the inner diameter of the stopper tube 12 and the outer diameter of the probe tube 11 is not greater than approximately 1 mm. By appropriately setting the inner diameter of the stopper tube 12 and the outer diameter of the probe tube 11, it is possible to more reliably fix the thermocouple conductive wires W1+ and W1-, and to crimp and electrically connect the high frequency conductive wire WO to the probe tube 11, as described later.

The material of the stopper tube 12 may be a resin material having flexibility and a certain degree of elasticity, and examples thereof include a fluororesin such as PTFE, FEP, and PFA, a fluororubber, a thermoplastic elastomer such as styrene, polyester, and nylon, a polyvinyl chloride, a polyolefin, and a silicone, and among these, a polyvinyl chloride (PVC) is preferable. The thickness and/or length of the stopper tube 12 may be adjusted to a thickness that can ensure the required strength and the length required for fixing the wire.

The pair of thermocouple conductive wires W1+ and W1- are led out from a proximal end opening portion of the probe tube 11, are folded back to the distal end side, and are sandwiched between the outer peripheral surface of the probe tube 11 and the inner peripheral surface of the stopper tube 12. As a result, a joint member such as a probe proximal part for fixing the thermocouple conductive wires W1+ and W1- is not required. The pair of thermocouple conductive wires W1+ and W1- sandwiched between the outer peripheral surface of the probe tube 11 and the inner peripheral surface of the stopper tube 12 appear on the distal end side of the stopper tube 12, and are further folded back to the proximal end side and are connected to the pair of compensation conductive wires W2+ and W2- by connection means 120 in the vicinity of the distal end portion of the medicinal liquid tube 16 on the proximal end side from the proximal end portion of the probe tube 11.

The connection means 120 is not particularly limited as long as the connection means 120 is known means that connects the wires to each other. Specific examples thereof include a connector, soldering, welding, and crimping, and among these, crimping using a crimp connector is preferable from the viewpoint of ease of assembly. The crimp connector is preferably a tubular member from the viewpoint that two wires can be easily connected, and the material thereof may be a conductive metal. Examples thereof include copper, aluminum, brass, and stainless steel (SUS), and among these, stainless steel is preferable from the viewpoint of corrosion resistance.

The pair of thermocouple conductive wires W1+ and W1- are electrically connected to each other at a temperature measurement contact point C1 disposed at the distal end portion of the probe tube 11. Each of the pair of thermocouple conductive wires W1+ and W1- and each of the pair of compensation conductive wires W2+ and W2- are electrically connected to each of a pair of compensation contact points C2+ and C2- connected by the connection means 120. The high frequency conductive wire WO and the pair of compensation conductive wires W2+ and W2- may be collectively accommodated in a wiring tube (not illustrated) having insulating properties and flexibility.

The probe tube 11 is made of, for example, a conductive material such as stainless steel. The high frequency conductive wire WO is made of, for example, a copper wire (for example, φ 0.18 mm) or an enameled wire. One thermocouple conductive wire W1+ and one compensation conductive wire W2+ are made of, for example, a chromel wire (for example, φ 0.1 mm). The other thermocouple conductive wire W1- and the other compensation conductive wire W2- are made of, for example, an alumel wire (for example, φ 0.1 mm). One compensation conductive wire W2+ may be made of a copper wire (for example, φ 0.1 mm), and the other compensation conductive wire W2- may be made of a constantan wire (for example, φ 0.1 mm). One thermocouple conductive wire W1+ may be made of a copper wire, and the other thermocouple conductive wire W1- may be made of a constantan wire. The high frequency conductive wire WO, the thermocouple conductive wires W1+ and W1-, and/or the compensation conductive wires W2+ and W2- may be covered with an insulating coating layer, except for a part constituting an electrical contact point.

As illustrated in FIG. 1, the high frequency electrode probe includes the high frequency conductive wire WO (refer to solid line), the pair of compensation conductive wires W2+ and W2- (refer to two-dot chain line), and the thermocouple connector 40. The high frequency conductive wire WO is sandwiched between the outer peripheral surface of the pair of thermocouple conductive wires W1+ and W1- of the probe tube 11 and the inner peripheral surface of the stopper tube 12, is crimped at this part to form the high frequency contact point C0, and is easily electrically connected to the probe tube 11 without passing through a soldering or another adhesion step. Therefore, the high frequency conductive wire WO and the insulating material of the probe tube 11 are not present in the vicinity of the high frequency contact point C0, and the underlying metal is exposed.

The thermocouple connector 40 includes a housing main body 420 and a pair of terminals 422 that protrude from the housing main body 420. The pair of terminals 422 are inserted into a terminal receiving portion having a high frequency power supply device (not illustrated) having a temperature measurement function to constitute reference temperature contact points C4+ and C4- electrically connected to the device (particularly, a temperature measurement device).

### (Configuration of Probe Tube)

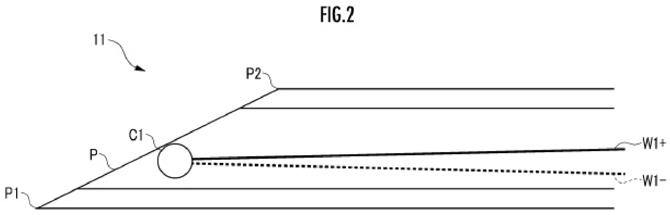

As illustrated in FIG. 2, the distal end portion of the probe tube 11 is formed with an inclined cutting edge surface P having a substantially planar shape and inclined with respect to the central axis of the probe tube 11. As illustrated in FIG. 2, the temperature measurement contact point C1, at which the pair of thermocouple conductive wires W1+ and W1- constituting the thermocouple 14 are electrically connected to each other, is disposed inside the probe tube 11 at a rear side of the inclined cutting edge surface P and midway between a distal end portion P1 and a proximal end portion P2 of the inclined cutting edge surface P. The temperature measurement contact point C1 may be disposed at a position corresponding to the proximal end portion P2 of the inclined cutting edge surface P or the rear side of the proximal end portion P2 of the inclined cutting edge surface P (for example, rear side by a distance equal to or less than 0.2 times the length of the inclined cutting edge surface P in an axial direction of the probe tube 11). The same applies to other embodiments.

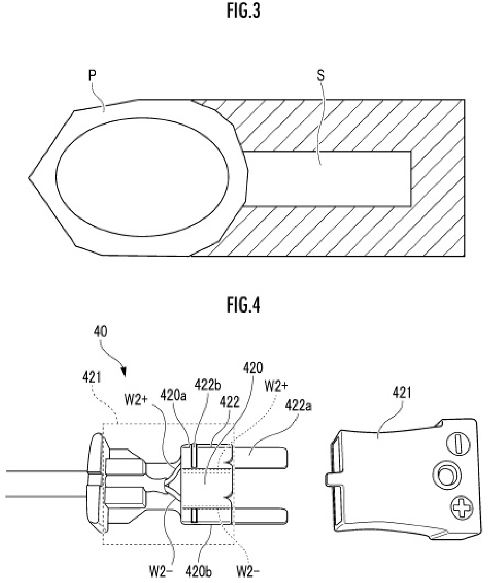

As shown in FIG. 3, the probe tube 11 is coated with an insulating material (for example, a fluorine coating material) except for the inclined cutting edge surface P and a local region S of the outer surface of the distal end portion. The local region S is a substantially rectangular region when viewed from a top, which extends in a range corresponding to the proximal end portion P2 of the inclined cutting edge surface P in a circumferential direction of the probe tube 11 on the outer surface of the distal end portion, and extends in the longitudinal direction (a direction parallel to the central axis) of the probe tube 11. The shape of the region may be variously changed, in addition to the substantially rectangular shape, such as a substantially triangular shape, a substantially trapezoidal shape, a substantially circular shape, a substantially oval shape, a substantially elliptical shape, or a combination of these shapes.

In another embodiment, the distal end portion of the probe tube 11 may be covered with an insulating material except for at least a part of the inclined cutting edge surface P, or the distal end portion of the probe tube 11 may be covered with an insulating material except for the entire circumference of the inclined cutting edge surface P and the outer surface of the distal end portion of the probe tube 11. The thickness of the probe tube 11 is not particularly limited, and examples thereof include 25G, 24G, 23G, 22G, 21G, 20G, 19G, 18G, and 17G in the gauge standard.

### (Configuration of Thermocouple Connector)

As schematically illustrated in FIG. 4, the thermocouple connector 40 includes the housing main body 420 and the pair of terminals 422 that protrude from the housing main body 420 in a substantially parallel direction. The terminals 422 are the pair of metal terminals 422 formed in an L shape by bending one end side of an elongated rectangular plate-like body by 90°, and includes a terminal main body 422a extending in the longitudinal direction and a fitting locking portion 422b (bent in a direction perpendicular to the paper plane in FIG. 4). The housing main body 420 has a horizontal fitting slit 420b cut from a side surface toward a center portion to have a depth corresponding to a width of the terminal 422, and a vertical fitting slit 420a cut to have the length corresponding to the width of the terminal 422 from an upper surface in the vicinity of a rear end portion (left side in the drawing) of the horizontal fitting slit 420b to reach the horizontal fitting slit 420b.

The terminal 422 is mounted on the horizontal fitting slit 420b from the side, and prior to this, each of the pair of compensation conductive wires W2+ and W2- are disposed in the horizontal fitting slit 420b. The terminal 422 is inserted toward the center of the housing main body 420 from the side of the horizontal fitting slit 420b, and in this case, the fitting locking portion 422b is inserted into the vertical fitting slit 420a. As a result, the terminal main body 422a is fitted and locked to the horizontal fitting slit 420b, and the fitting locking portion 422b is fitted and locked to the vertical fitting slit 420a, and thus the terminal main body 422a and the fitting locking portion 422b are fixed. In addition, at the same time, the pair of compensation conductive wires W2+ and W2-disposed in the horizontal fitting slit 420b are crimped to each of the terminals 422 and electrically connected. Finally, the thermocouple connector 40 is formed by disposing a cover 421 at the position of the broken line. As described above, the thermocouple connector 40 can be easily manufactured only by fitting the compensation conductive wires W2+ and W2- and the pair of terminals 422 to the housing main body 420, and the workability is improved. In addition, the same applies to a case where the thermocouple conductive wires W1+ and W1- are used instead of the compensation conductive wires W2+ and W2-, as described later.

### (Second Embodiment of Present Invention)

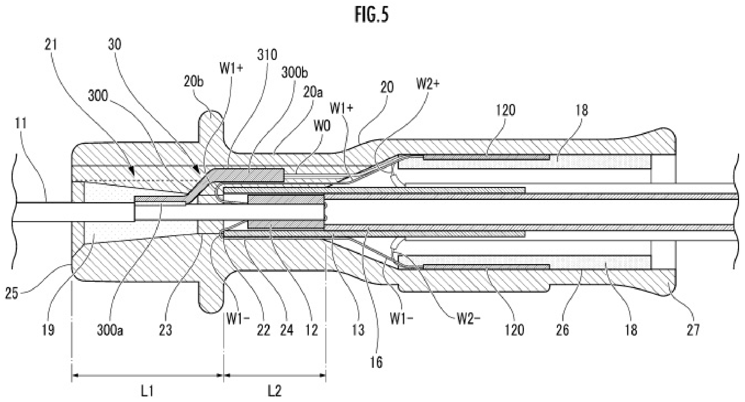

FIG. 5 illustrates a cross-sectional view of a configuration (X2 part in FIG. 1) in the vicinity of a probe proximal cover 20 of a high frequency electrode probe according to a second embodiment of the present invention. The configuration of other parts is the same as that in FIG. 1, and the same components as those in FIG. 1 are denoted by the same reference numerals, and the description thereof will be omitted. The second embodiment differs from the first embodiment mainly in that the probe proximal cover 20 that encloses the proximal end portion of the probe tube 11 on the proximal end portion side of the probe tube 11 and serves as a handle of the probe tube 11 is provided, and an indexing mechanism 30 that can mechanically regulate the circumferential position of the cutting edge surface of the probe tube 11 with respect to the circumferential direction of the probe proximal cover 20 is provided.

As illustrated in FIG. 5, the probe proximal cover 20 is a hollow cylindrical member, and a constricted portion 20a is formed on a central distal end side thereof, and a flange-shaped protruding portion 20b is formed on a distal end side of the constricted portion 20a. By forming the constricted portion 20a and the protruding portion 20b, it is easy for the operator to hold. In addition, a small diameter portion 23 having the smallest diameter is formed on the inner peripheral surface corresponding to the protruding portion 20b, and a cylindrical probe tube proximal end storage portion 24 having a diameter larger than the diameter of the small diameter portion 23 is formed in the direction from the small diameter portion 23 to the proximal end portion 27 through a stepped portion 22. The stopper tube 12 mounted on the probe tube proximal end portion 11a of the probe tube 11 is stored and disposed in an internal space of the probe tube proximal end storage portion 24. The medicinal liquid tube 16 is connected to the proximal end portion of the stopper tube 12. Examples of materials of the probe proximal cover 20 include an ABS resin, polypropylene (PP), polycarbonate (PC), polyvinyl chloride (PVC), and the like.

The connection tube 13 is tightly and externally attached to the outer periphery of the stopper tube 12 and the outer periphery of the distal end portion of the medicinal liquid tube 16. As a result, the proximal end portion of the stopper tube 12 and the distal end portion of the medicinal liquid tube 16 are liquid-tightly connected to each other, and the outer periphery of the stopper tube 12 and the outer periphery of the medicinal liquid tube 16 are liquid-tightly connected to each other. Therefore, the difference between the inner diameter of the connection tube 13 and the outer diameter of the stopper tube 12 and the medicinal liquid tube 16 is preferably at least 0.01 mm or more, and more preferably 0.05 mm or more. The upper limit thereof is preferably 0.15 mm and more preferably 0.1 mm. Examples of materials of the connection tube 13 include the same material as that of the stopper tube 12, and the thickness and/or the length thereof may be set to a thickness and/or the length at which the probe tube 11 and the medicinal liquid tube 16 can be appropriately connected and maintained.

As described above, the stepped portion 22 is formed between the small diameter portion 23 of the inner peripheral surface of the probe proximal cover 20 and the probe tube proximal end storage portion 24. The distal end of the connection tube 13 abuts on the stepped portion 22, so that the stepped portion 22 regulates the position of the probe tube 11 in the longitudinal direction. That is, the connection tube 13 functions as a positioning tube. Therefore, the positioning structure including the stepped portion 22 and the distal end portion of the connection tube 13 functions as a cannula stop that defines the effective length of the probe tube 11. That is, the effective length of the probe tube 11 is the length from a distal end 25 of the probe proximal cover 20 to a probe distal part P (P1), but the actual length of the probe tube 11 is the length obtained by adding the length L1 + L2 of the probe tube 11 hidden inside the probe proximal cover 20 from the distal end 25 of the probe proximal cover 20 to the effective length. Therefore, when the distal end of the connection tube 13 abuts on the stepped portion 22 in the probe proximal cover 20, the effective length of the probe is automatically determined. The position of the distal end of the connection tube 13 may be adjusted as necessary to adjust the effective length of the probe tube 11.

As described above, by causing the stepped portion 22 of the inner peripheral surface of the probe proximal cover 20 to function as the cannula stop of the probe tube 11, the same action and effect as the cannula stop of the probe tube 11 can be obtained even in a configuration in which the probe proximal part, which is the connection joint, is not used. In addition, it is not necessary to set the inner diameter of the cannula stop for each size of the probe, the manufacturing device is simplified, manufacturing is facilitated, cost can be reduced, and the problem of a sink mark can be solved.

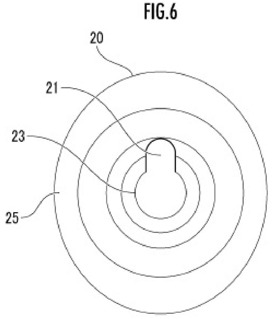

In this example, the indexing mechanism 30 is a fitting mechanism in which a protrusion structure 300 formed to protrude in the radial direction at a circumferential fixed position on the outer peripheral surface of the probe tube 11, and a recessed structure 21 formed at a circumferential fixed position on the inner peripheral surface of the probe proximal cover 20 are fitted to be fixed, as illustrated in FIGS. 5 and 6. In addition, the indexing mechanism 30 may have a structure in which the shape of the proximal end portion of the probe tube 11 is rotationally asymmetric with the longitudinal axis as the rotation center and the inner periphery of the probe proximal cover 20 has a shape corresponding to the shape of the proximal end portion of the probe tube 11, such that the two are fitted into each other only at a specific circumferential position.

The protrusion structure 300 formed on the probe tube 11 is not particularly limited, and in this example, a metal pipe 310 formed in a crank shape or a substantially Z shape is fixed to a fixed position on the peripheral surface of the probe tube 11. As described above, by fixing one end side of the metal pipe 310, which is alternately bent at two bending points to be formed in a crank shape or a Z shape, to the probe tube 11, a fitting projecting portion 300b that is spaced apart from or protrudes from the probe tube 11 in the circumferential direction can be formed, and the fitting projecting portion 300b functions as the protrusion structure 300. In addition, the protrusion structure 300 is not limited to the above-described example, and for example, a three-dimensional shape such as a hemisphere, a cylinder, a cube, or a rectangular parallelepiped may protrude from the outer peripheral surface of the probe tube 11 in the radial direction. A fitting mechanism may be provided by preparing a recessed shape corresponding to the protrusion structure 300.

The advantage of using the metal pipe 310 for the protrusion structure 300 is that, as described above, a commercially available metal pipe 310 can be used, and the protrusion structure 300 can be easily formed simply by bending processing. In addition, the protrusion structure 300 can also function as a fixing and connecting structure for a high frequency conductive wire. That is, the high frequency conductive wire WO is passed through the metal pipe 310 until the distal end thereof is slightly exposed, one end side fixed to the probe tube 11 is crushed, and the high frequency conductive wire WO is crimped and connected to be electrically conductive to the metal pipe 310. The high frequency conductive wire WO exposed from the distal end is cut, bent as described above, and then fixed to the probe tube 11 by a fixing portion 300a. Examples of means for forming the fixing portion 300a include a method of fixing while achieving electrical conduction, such as welding, soldering, brazing, and screwing, and a suitable one may be appropriately selected from these. As described above, by electrically connecting the high frequency conductive wire WO to the probe tube 11 by the metal pipe 310, the metal pipe 310 receives a stress load when the probe tube 11 is inserted into the probe proximal cover 20, and the risk of disconnection of the high frequency conductive wire WO is reduced, and the quality can be stabilized.

Since the indexing mechanism 30 mechanically determines the position of the cutting edge surface of the probe tube 11 in the circumferential direction with respect to the circumferential direction of the probe proximal cover 20, the assembly work is facilitated. In addition, the operator can confirm the position of the cutting edge surface by looking at the probe proximal cover 20 at hand, and the workability of the treatment is improved. In this case, the probe proximal cover 20 may be provided with a mark indicating the position of the cutting edge surface. As the mark, in addition to a pattern such as a character or a symbol, a shape feature such as a protrusion or a recessed portion may be provided. The probe tube 11 may be stably fixed by filling an internal space from the small diameter portion 23 to the distal end 25 of the probe proximal cover with an adhesive 19a as necessary. The configuration in which the probe proximal cover 20 and the connection tube 13 are provided is also preferably applied to the first embodiment.

Even in the present embodiment illustrated in FIG. 5, the pair of thermocouple conductive wires W1+ and W1- led from the proximal end opening portion of the probe tube 11 are folded back to the distal end side, and are sandwiched between the outer peripheral surface of the probe tube proximal end portion 11a of the probe tube 11 and the inner peripheral surface of the stopper tube 12. The pair of thermocouple conductive wires W1+ and W1- appear at the distal end side of the stopper tube 12, are further folded back toward the proximal end side, and are connected to the pair of compensation conductive wires W2+ and W2- by the connection means 120 at a position enclosed in the probe proximal cover 20 on the proximal end side from the probe tube proximal end portion 11a of the needle tube 11, that is, around the distal end portion of the medicinal liquid tube 16. As the connection means 120, a crimp connector is preferable, and a tubular stainless steel is more preferable.

In the present embodiment, the high frequency conductive wire WO is connected to one of the pair of compensation conductive wires W2+ and W2- by the connection means 120. That is, one of the pair of compensation conductive wires W2+ and W2-functions as a high frequency conductive wire between the connection means 120 and the thermocouple connector 40. In this case, it is preferable that one compensation conductive wire W2+ of the pair of compensation conductive wires W2+ and W2- is made of a copper wire (for example, φ 0.1 mm), and the other compensation conductive wire W2- may be made of a constantan wire (for example, φ 0.1 mm).

As described above, by causing one of the pair of compensation conductive wires W2+ and W2- to function as the high frequency conductive wire, the wiring to the thermocouple connector 40 can be reduced, the risk of disconnection can be reduced, the manufacturing is facilitated, and the cost can be reduced.

In the present embodiment, the pair of compensation conductive wires W2+ and W2- and the medicinal liquid tubes 16 to which the connection tube 13 is externally attached are bundled by a binding tube 18. Each strand of the pair of compensation conductive wires W2+ and W2- may be covered with an internal coating material and may be integrally coated with an external coating material. The binding tube 18 enclosing the contents is stored and fixed in a binding tube storage portion 26 in which the inner peripheral surface of the probe proximal cover 20 is larger in diameter than the probe tube proximal end storage portion 24. In this case, since the outer diameter of the binding tube 18 enclosing the contents is set to be larger than the inner diameter of the binding tube storage portion 26, the stored binding tube 18 is compressed such that the outer peripheral surface thereof is pressed against the inner peripheral surface of the binding tube storage portion 26 and fixed.

Furthermore, the crimp connector, which is the connection means 120, is also sandwiched and fixed between the outer peripheral surface of the binding tube 18 and the inner peripheral surface of the binding tube storage portion 26. The material of the binding tube 18 may also be the same as that of the stopper tube 12, and the length and/or thickness thereof may be adjusted to an appropriate length and/or thickness inside the probe proximal cover 20. The binding tube 18 is preferably formed in a C-shaped cross section in which one side surface of a cylindrical tube body is cut. By forming the binding tube 18 in this manner, the content can be inserted from the opening of the side surface and mounted, and the workability is improved.

### (Third Embodiment of Present Invention)

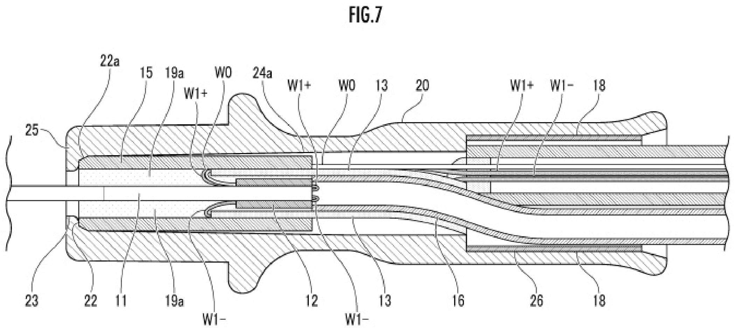

FIG. 7 illustrates a cross-sectional view of a configuration (X2 part in FIG. 1) in the vicinity of a probe proximal cover 20 of a high frequency electrode probe according to a third embodiment of the present invention. The main difference between the third embodiment and the second embodiment is that the indexing mechanism 30 is not provided and the thermocouple conductive wires W1+ and W1- are directly electrically connected to the thermocouple connector 40 without using the compensation conductive wires W2+ and W2-. The same reference numerals are assigned to the same configurations as those in the second embodiment, and the description thereof will be omitted.

Since the high frequency electrode probe illustrated in FIG. 7 does not have the indexing mechanism, a probe tube proximal end storage portion 24a having an inner diameter slightly increased in diameter from the distal end toward the proximal end side in the axial direction is formed in the distal end side inner diameter of the probe proximal cover 20, and a binding tube storage portion 26 having a larger diameter than the probe tube proximal end storage portion 24a is formed on the proximal end portion side. In addition, at the distal end portion of the probe tube proximal end storage portion 24a, the stepped portion 22 that rises in the axial direction from an inclined surface 22a inclined in the distal end direction so that the diameter decreases slightly toward the central axis direction, and the small diameter portion 23 that is open to a diameter smaller than that of the probe tube proximal end storage portion 24a from the stepped portion 22 to the probe proximal cover distal end 25 are formed. A chamfered portion having a rounded shape is formed at an inner end portion of the small diameter portion 23. A centering tube 15 is further externally attached to the outer periphery of the stopper tube 12 to which the connection tube 13 is externally attached, that is, the outer periphery of the connection tube 13. The centering tube 15 is stored in the probe tube proximal end storage portion 24a, and the outer peripheral surface of the distal end portion thereof abuts on the inclined surface 22a and is compressed, such that the probe tube 11 to which the centering tube 15 is externally attached is positioned at the radial center. An internal space from the distal end side inside the centering tube 15 to the distal end of the stopper tube 12 may be filled with the adhesive 19a as necessary to stably fix the probe tube 11.

In the third embodiment, the centering tube 15 functions as a positioning tube. That is, the distal end of the centering tube 15 abuts on the stepped portion 22 to regulate the axial position of the probe tube 11. Therefore, the effective length of the probe tube 11 is the length from the distal end of the probe proximal cover 20 to the distal end P of the probe tube 11, and the actual length of the probe tube 11 is the length obtained by adding the length of the probe tube 11 hidden inside the probe proximal cover 20 from the probe proximal cover distal end 25 to the effective length. Here, the probe tube proximal end portion 11a of the probe tube 11, the proximal end portion of the stopper tube 12, and the proximal end portion of the connection tube 13 may be aligned at the same position in the axial direction. By aligning these at the same position, it is easy to adjust the effective length. In this case, for example, by coloring the stopper tube l2 and the connection tube 13, visibility is improved, and the stopper tube 12 and the connection tube 13 are easily aligned at the same position. The position of the distal end of the connection tube 13 may be adjusted as necessary to adjust the effective length of the probe tube 11.

In the third embodiment, in order to position the position of the cutting edge surface of the probe tube 11 with respect to the probe proximal cover 20, a jig capable of mechanically matching a circumferential specific position of the probe tube 11 and a circumferential specific position of the probe proximal cover 20 may be used. By using the jig, the probe tube 11 and the probe proximal cover 20 are easily aligned in the circumferential direction, and productivity is improved.

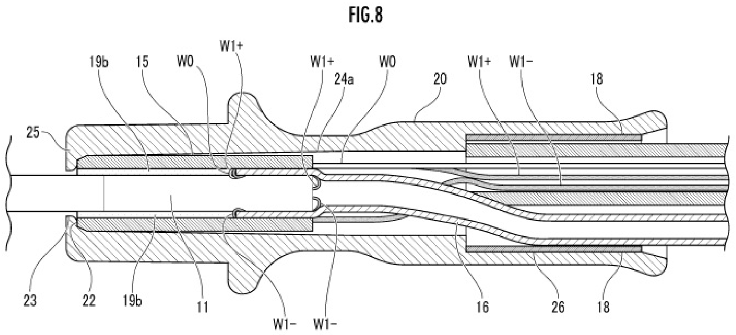

FIG. 8 illustrates a modification example of the third embodiment. In this example, in order to use the large-diameter probe tube 11, the stopper tube 12 is omitted, and the distal end portion of the medicinal liquid tube 16 is externally attached to the proximal end portion of the probe tube 11 instead of the stopper tube 12. The thickness of the probe tube 11 in the exemplified aspect is not particularly limited, but for example, a probe tube 11 having a thickness of 20 G or more is preferable, and particularly 19 G, 18 G, 17 G, or the like is preferable. An internal space from the distal end side inside the centering tube 15 to the distal end of the medicinal liquid tube 16 may be filled with an adhesive 19b as necessary to stably fix the probe tube 11. In addition, the internal space may be widened by providing the connection tube 13 between the centering tube 15 and the medicinal liquid tube 16, and the inflow of the adhesive 19b may be improved.

Each component described in the first to third embodiments is not limited to a specific embodiment and can be freely used between each of the embodiments as necessary. For example, in the first embodiment, the probe proximal cover 20 may be used, and in the third embodiment, the compensation conductive wires W2+ and W2-may be connected to the thermocouple conductive wires W1+ and W1- via the connection means 120.

### Industrial Applicability

The high frequency electrode probe according to the configuration can directly administer a local anesthetic to a peripheral nerve (a spinal nerve or a sympathetic ganglion) or in the vicinity thereof in the medical field, can destroy the nerve, and can stop the nerve function temporarily or for a long period of time to reduce pain by performing high frequency thermal coagulation and pulsed high frequency electrical stimulation. Furthermore, the high frequency electrode probe can be applied to sympathetic nerve blocks to improve blood flow not only in diseases that cause pain, but also in diseases that do not cause pain, such as facial nerve paralysis, central retinal artery occlusion, allergic rhinitis, and sudden hearing loss.

### Description of Reference Numerals

11: probe tube
11a: probe tube proximal end portion
12: stopper tube
13: connection tube
14: thermocouple
15: centering tube
16: medicinal liquid tube
18: binding tube
20: probe proximal cover
20a: constricted portion
20b: protruding portion
22: stepped portion
22a: inclined surface
23: small diameter portion
24: probe tube proximal end storage portion
25: probe proximal cover distal end
26: binding tube storage portion
300: protrusion structure
310: metal pipe
40: thermocouple connector
420: housing main body
422: terminal
420a: vertical fitting slit
420b: horizontal fitting slit
421: cover
422a: terminal main body
422b: fitting locking portion
C1: temperature measurement contact point
C2: compensation contact point
C4: reference temperature contact point
P: inclined cutting edge surface
WO: high frequency conductive wire
W1+, W1-: thermocouple conductive wire
W2+, W2-: compensation conductive wire

## Claims

1. A high frequency electrode probe with a temperature measurement function, comprising:
a cylindrical probe tube;
a thermocouple including a pair of thermocouple conductive wires electrically connected at a temperature measurement contact point disposed at a distal end portion of the probe tube; and
a high frequency conductive wire electrically connected to the probe tube,
wherein a stopper tube having flexibility is externally attached to a probe tube proximal end portion serving as a proximal end portion of the probe tube, and
the thermocouple conductive wire is sandwiched between an outer peripheral surface of the probe tube and an inner peripheral surface of the stopper tube.

2. A high frequency electrode probe with a temperature measurement function, comprising:
a cylindrical probe tube;
a thermocouple including a pair of thermocouple conductive wires electrically connected at a temperature measurement contact point disposed at a distal end portion of the probe tube;
a high frequency conductive wire electrically connected to the probe tube; and
a medicinal liquid tube configured to supply a medicinal liquid to the probe tube,
wherein the thermocouple conductive wire is sandwiched between an outer peripheral surface of the probe tube and an inner peripheral surface of the medicinal liquid tube.

3. The high frequency electrode probe with a temperature measurement function according to Claim 1 or 2, further comprising:
a probe proximal cover configured to enclose the probe tube proximal end portion and serve as a handle of the probe tube at the proximal end portion of the probe tube.

4. The high frequency electrode probe with a temperature measurement function according to Claim 3, further comprising:
an indexing mechanism configured to mechanically regulate a circumferential position of a cutting edge surface of the probe tube with respect to a specific position of the probe proximal cover in a circumferential direction.

5. The high frequency electrode probe with a temperature measurement function according to Claim 4,
wherein the indexing mechanism is a fitting mechanism of a protrusion structure formed at a circumferential fixed position of the probe tube and a recessed structure formed at a fixed position on an inner peripheral surface of the probe proximal cover.

6. The high frequency electrode probe with a temperature measurement function according to Claim 5,
wherein in the protrusion structure, a metal pipe formed in a crank shape or a Z shape is fixed to the circumferential fixed position on an outer periphery of the probe tube.

7. The high frequency electrode probe with a temperature measurement function according to Claim 6,
wherein the high frequency conductive wire is crimped and connected within the metal pipe of the protrusion structure and is electrically conductive to the probe tube via the metal pipe.

8. The high frequency electrode probe with a temperature measurement function according to Claim 1,
wherein the high frequency conductive wire is sandwiched between the outer peripheral surface of the probe tube and the inner peripheral surface of the stopper tube, and is crimped and connected to the probe tube to be electrically conductive.

9. The high frequency electrode probe with a temperature measurement function according to Claim 2,
wherein the high frequency conductive wire is sandwiched between the outer peripheral surface of the probe tube and the inner peripheral surface of the medicinal liquid tube, and is electrically conductive by being crimped and connected to the probe tube.

10. The high frequency electrode probe with a temperature measurement function according to Claim 1 or 2,
wherein the thermocouple conductive wire extends to and is directly electrically connected to a thermocouple connector for connecting to a high frequency power supply device without passing through a compensation conductive wire.

11. The high frequency electrode probe with a temperature measurement function according to Claim 1 or 2,
wherein the thermocouple conductive wire is connected to a compensation conductive wire on a proximal end side from the proximal end portion of the probe tube by a crimp connector, and the compensation conductive wire extends to a thermocouple connector for connecting to a high frequency power supply device.

12. The high frequency electrode probe with a temperature measurement function according to Claim 11,
wherein the high frequency conductive wire is connected by the crimp connector to one of the compensation conductive wires together with one of the thermocouple conductive wires.

13. The high frequency electrode probe with a temperature measurement function according to Claim 3,
wherein a positioning tube is externally attached to the probe tube proximal end portion, and a distal end portion of the positioning tube is locked to a stepped portion inside the probe proximal cover to regulate an axial position of the probe tube.
